# EUROPEAN PATENT SPECIFICATION

(11) **EP 1 219 316 B1**
(45) Date of publication and mention of the grant of the patent: **04.07.2007**
(21) Application number: 01309645.8
(22) Date of filing: 15.11.2001
(51) Int. Cl.: A61M 16/04

(54) **Method of forming a laryngeal mask assembly**
Verfahren zur Herstellung einer Kehlkopfmaske
Procédé de fabrication d'ensemble de masque laryngé

(30) Priority: 22.12.2000 GB 0031661
(43) Date of publication of application: 03.07.2002
(73) Proprietor: Smiths Group plc, London, NW11 8DS (GB)
(72) Inventor: Collins, Michael Norman, Lyminge, Folkestone, Kent CT18 8HD (GB); Pagan, Eric, Kent CT21 8BD (GB)
(74) Representative: Flint, Jonathan McNeill

(56) References cited:
- EP-A- 0 922 465
- EP-A- 1 125 595
- WO-A-00/61213
- WO-A1-01/13979
- WO-A1-01/13980
- WO-A2-20/04089453
- GB-A- 2 334 215
- GB-A- 2 354 950
- GB-A- 2 373 188
- US-A- 4 509 514
- US-A- 5 241 956
- US-A- 5 540 224
- US-A- 5 771 889
- US-A- 5 871 012
- US-A- 5 881 726
- US-A- 5 979 445
- US-A- 5 983 897
- US-A- 6 003 514
- US-A- 6 012 452
- US-A- 6 021 779
- US-A- 6 050 264
- US-A- 6 095 144
- US-A- 6 116 243
- US-B1- 6 240 922
- US-B1- 6 261 401
- ANDRE A. ET AL: 'Comparison of the LMA-Classic with the New Disposable Soft Seal Laryngeal Mask in Spontaneously Breathing Adult Patients' ANESTESIOLOGY vol. 99, no. 5, November 2003, pages 1066 - 1071

## Description

This invention relates to methods of forming laryngeal mask assemblies.

It is common practice to use an airway known as a laryngeal mask for administering anaesthetic and ventilation gases to a patient. These airways comprise a tube with an inflatable mask or cuff at one end, the tube being inserted in the patient's mouth so that one end is located in the hypopharynx and so that the mask forms a seal in this region with the surrounding tissue. Laryngeal masks are described in, for example, US 5355879, US 5305743, US 5297547, US 5282464, GB 2267034, US 5249571, US 5241956, US 5303697, GB 2249959, GB 2111394, EP 448878, US 4995388, GB 2205499, GB 2128561, GB 2298797, GB 2334215, GB2337020, PCT/GB00/03044, PCT/GB00/03045, GB 0002805 and GB 0020274. Laryngeal masks usually comprise a curved, extruded tube, a separate mount member joined at the patient end of the tube and an inflatable cuff attached to the mount member. WO 00/61213 describes a laryngeal mask where the tube and mount member are made together by rotational moulding.

Laryngeal masks have several advantages over endotracheal tubes, which are longer and seal with the trachea below the vocal folds. The multiple components and assembly operations needed to make the masks, however, add to their cost.

It is an object of the present invention to provide an alternative method of forming a laryngeal mask assembly.

According to the present invention there is provided a method of forming a laryngeal mask assembly including the steps of moulding a first integral component comprising a tube and a mount with the mount at the patient end of the tube, forming a second component in the form of an inflatable annular sealing cuff entirely of a thin, flexible, inflatable plastics material and having a circular transverse section throughout, and attaching the thin, flexible, inflatable, circular section of the cuff directly with the mount so that the cuff extends around the patient end of the mount.

The sealing cuff may be attached with the mount by an adhesive. The tube and mount are preferably moulded together by injection moulding and may be moulded of polyurethane

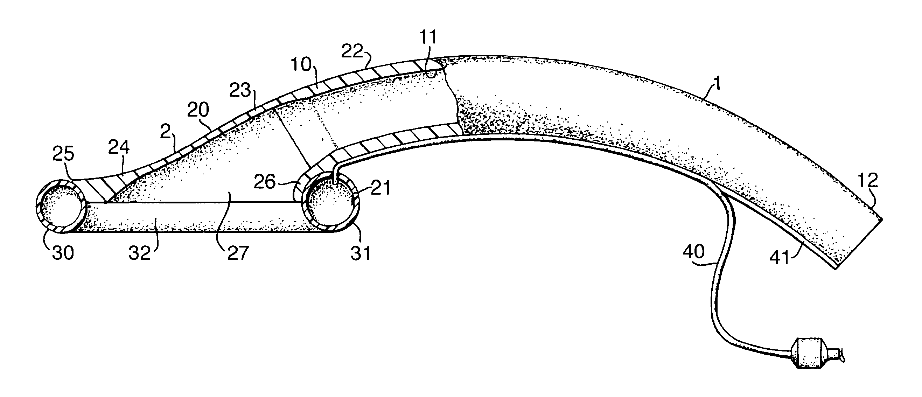

A laryngeal mask assembly according to the present invention will now be described, by way of example, with reference to the accompanying drawing, which is a side elevation view of the assembly.

The laryngeal mask assembly comprises a tube 1 and a mask formation 2 at the patient end 10 of the tube.

The tube 1 is of a bendable plastics material, such as PVC and is curved along its length. A bore 11 extends along the tube from its patient end 10 to its rear, machine end 12.

The mask 2 comprises a mount 20 and an inflatable sealing cuff 21. The mount 20 is of a relatively stiff plastics material and is of generally shoe shape. The mount 20 and tube 1 are moulded together, such as by injection moulding, to form an integral, single piece 22. The mount 20 tapers outwardly from its machine end 23 to its patient end 24, which is inclined to the axis of the machine end at an angle of about 25° so that the patient end of the mount has an oval shape with its forward end 25 being more pointed than its rear end 26. The patient end 24 of the mount 20 is inclined to face towards the inner side of the curve of the tube 1. Internally, the mount 20 has a cavity 27 that increases in cross-sectional area along its length, from the machine end.

The cuff 21 is tubular and of a thin flexible plastics material. The cuff 21 is formed into an annulus of the same shape as the patient end 24 of the mount 20 so that it is oval with its forwardly-directed end 30 being more pointed than its rearwardly-directed end 31. The cuff 21 encloses a central region 32 of the same shape as the patient end 24 of the mount 20. The cuff 21 is attached around the patient end 24 of the mount 20 such as by means of an adhesive. The cuff 21 is inflated and deflated by means of an inflation line 40 which is provided by a separate small-bore tube communicating with the interior of the cuff and extending rearwardly along a groove 41 in the outside of the tube. When inflated in position in a patient, the cuff 21 expands to contact patient tissue in the region of the hypopharnyx.

Because the mount and tube are formed in one operation, there are fewer separate components and fewer steps needed to manufacture the assembly. By avoiding the need to bond the mount to the tube, there is no risk of a faulty bond and there is no need to inspect a bond. It is possible to achieve a smooth external profile in the region between the tube and mount, without a step or bump, because there is no connection in this region. Moulding the tube and mount together enables the wall thickness or shape to be varied, if desired, at different points along the length of the tube. It is also possible, by moulding, to use different materials, such as polyurethane, which present problems with extrusion.

## Claims

1. A method of forming a laryngeal mask assembly including the steps of moulding a first integral component comprising a tube (1) and a mount (20) with the mount at the patient end of the tube, forming a second component in the form of an inflatable annular sealing cuff (21) entirely of a thin, flexible, inflatable plastics material, **characterized by** attaching the thin, flexible, inflatable, circular section of the cuff (21) directly with the mount (20) so that the cuff extends around the patient end of the mount, and by that said cuff (21) has a circular transverse section throughout.

2. A method of forming a laryngeal mask assembly according to Claim 1, **characterised in that** the sealing cuff (21) is attached with the mount (20) by an adhesive.

3. A method according to Claim 1 or 2, **characterised in that** the tube (1) and mount (20) are moulded together by injection moulding.

4. A method of forming a laryngeal mask assembly according to any one of the preceding claims, **characterised in that** the tube (1) and mount (20) are moulded of polyurethane.

## Patentansprüche

1. Verfahren zum Ausbilden einer Kehlkopfmaskenanordnung mit den Schritten des Formens einer ersten einteiligen Komponente mit einem Tubus (1) und einer Halterung (20), wobei sich die Halterung am Patientenende des Tubus befindet, des Ausbildens einer zweiten Komponente in Form einer aufblasbaren, ringförmigen Dichtungsmanschette (21), die vollständig aus einem dünnen, biegsamen, aufblasbaren Kunststoffmaterial besteht, **gekennzeichnet durch** Befestigen des dünnen, biegsamen, aufblasbaren kreisförmigen Abschnitts der Manschette (21) direkt an der Halterung (20), so dass sich die Manschette um das Patientenende der Halterung erstreckt, und **dadurch**, dass die Manschette (21) durchweg einen kreisförmigen Querschnitt aufweist.

2. Verfahren zum Ausbilden einer Kehlkopfmaskenanordnung nach Anspruch 1, **dadurch gekennzeichnet, dass** die Dichtungsmanschette (21) durch einen Klebstoff an der Halterung (20) befestigt wird.

3. Verfahren nach Anspruch 1 oder 2, **dadurch gekennzeichnet, dass** der Tubus (1) und die Halterung (20) durch Spritzgießen zusammen geformt werden.

4. Verfahren zum Ausbilden einer Kehlkopfmaskenanordnung nach einem der vorangehenden Ansprüche, **dadurch gekennzeichnet, dass** der Tubus (1) und die Halterung (20) aus Polyurethan geformt werden.

## Revendications

1. Procédé de fabrication d'un ensemble de masque laryngé comprenant les étapes de moulage d'un premier composant réalisé d'une pièce comprenant un tube (1) et une monture (20) avec la monture disposée à l'extrémité patient du tube, de formation d'un second composant sous la forme d'une manchette d'obturation annulaire gonflable (21) entièrement réalisée en un matériau plastique gonflable, flexible et mince, **caractérisé par le fait qu'**on lie la section circulaire, gonflable flexible et mince de la manchette (21) directement à la monture (20) pour que la manchette s'étende autour de l'extrémité patient de la monture, et **par le fait que** ladite manchette (21) comporte une section transversale circulaire.

2. Procédé de fabrication d'un ensemble de masque laryngé selon la revendication 1, **caractérisé en ce que** la manchette d'obturation (21) est liée à la monture (20) par un adhésif.

3. Procédé selon la revendication 1 ou 2, **caractérisé en ce que** le tube (1) et la monture (20) sont moulés ensemble par moulage par injection.

4. Procédé de fabrication d'un ensemble de masque laryngé selon l'une quelconque des revendications précédentes, **caractérisé en ce que** le tube (1) et la monture (20) sont moulés en polyuréthane.
